# EUROPEAN PATENT APPLICATION

(11) **EP 2 682 060 A1**
(43) Date of publication of application: **08.01.2014**
(21) Application number: 13166730.5
(22) Date of filing: 07.05.2013
(51) Int. Cl.: A61B 8/00

(54) **Method and apparatus for displaying three-dimensional ultrasonic image and two-dimensional ultrasonic image**

(30) Priority: 11.06.2012 KR 20120062349
(71) Applicant: Samsung Medison Co., Ltd., Gangwon-do 250-870 (KR)
(72) Inventor: Park, Sung-wook, Hongcheon-gun, Gangwon-do (KR); Lee, Jin-yong, Hongcheon-gun, Gangwon-do (KR)
(74) Representative: Lorenz, Markus

(57) **Abstract**

A method of displaying a 3D image and a 2D image includes the operations of acquiring the 3D image of a target object, selecting at least one cross-section of the target object based on an external input for the acquired 3D image, acquiring the 2D image by scanning the target object such as to obtain the selected at least one cross-section, and displaying the 2D image and the 3D image.

## Description

### CROSS-REFERENCE TO RELATED PATENT APPLICATION

This application claims the benefit of Korean Patent Application No. 10-2012-0062349, filed on June 11, 2012, in the Korean Intellectual Property Office, the disclosure of which is incorporated herein in its entirety by reference.

### BACKGROUND OF THE INVENTION

### 1. Field of the Invention

The present invention relates to a method and apparatus for displaying a three-dimensional (3D) ultrasonic image and a two-dimensional (2D) ultrasonic image, and more particularly, to a method and apparatus for acquiring a 2D ultrasonic image by scanning a target object in the direction of a cross-section selected from an acquired 3D ultrasonic image, and displaying the acquired 3D and 2D ultrasonic images.

### 2. Description of the Related Art

Ultrasonic diagnosis apparatuses transmit an ultrasonic signal toward a predetermined part of the inside of a target object through the body surface of the target object and obtain an image associated with the fault or blood flow of soft tissue of the target object by using information of the ultrasonic signal reflected from tissue of the inside of the target object.

Such ultrasonic diagnosis apparatuses are capable of displaying an image of a target object in real time. In addition, such ultrasonic diagnosis apparatuses are very safe because there is no radiation exposure by X rays or the like, and thus are widely used together with other image diagnosis apparatuses, such as an X-ray diagnosis apparatus, a computed tomography (CT) scanner, a magnetic resonance imaging (MRI) apparatus, and a nuclear medicine diagnosis apparatus.

Three-dimensional (3D) ultrasonic images facilitate general and clear understanding of a target object. However, using two-dimensional (2D) ultrasonic images may be more effective than using 3D ultrasonic images, in order to observe the cross-section or the like of the inside of a target object in detail.

Accordingly, when a 3D ultrasonic image and a 2D ultrasonic image are displayed together, a user is able to observe a target object generally and minutely at the same time.

In the prior art, a 2D ultrasonic image of a target object is acquired from a 3D ultrasonic image of the target object by using a rendering technique. However, direct scanning of a target object instead of rendering may increase the frame rate of an image and may ensure high-quality images. Direct scanning of a target object may also enable real-time understanding of movement conditions and the like of a target object that moves a lot or moves fast.

### SUMMARY OF THE INVENTION

The present invention provides a method and apparatus for displaying a three-dimensional (3D) ultrasonic image and a two-dimensional (2D) ultrasonic image.

According to an aspect of the present invention, there is provided an apparatus for displaying a 3D ultrasonic image and a 2D ultrasonic image, the apparatus comprising: a 3D image acquiring unit which acquires a 3D image of a target object; a cross-section selection unit which selects at least one cross-section of the target object based on an external input for the acquired 3D image; a 2D image acquiring unit which acquires a 2D image by scanning the target object such as to obtain the selected at least one cross-section; and a display unit which displays the 2D image and the 3D image.

The 3D image acquiring unit may acquire the 3D image of the target object by combining a plurality of pieces of image data that are acquired for each cycle of an ultrasound having a predetermined period.

The cross-section selection unit may comprise a window producing unit which produces at least one window which is to be located on the acquired 3D image, and a window control unit which moves the at least one window produced on the 3D image.

The at least one cross-section may be selected from the 3D image of the target object by the cross-section selection unit so as to correspond to the location of the at least one window moved on the 3D image.

The 2D image acquiring unit may acquire at least one 2D image by scanning the target object in the scan direction of the selected at least one cross-section.

The display unit may display the 2D image and the 3D image.

The cross-section selection unit may further comprise an additional cross-section selection unit which additionally selects at least another cross-section adjacent to the selected at least one cross-section.

The adjacent at least one cross-section may be a cross-section that is a predetermined distance separated from the at least one cross-section selected from the 3D image.

The 2D image acquiring unit may comprise a first image acquiring unit which acquires at least one first 2D image by scanning the target object such as to obtain the selected at least one cross-section, a second image acquiring unit which acquires at least one second 2D image by scanning the target object such as to obtain the at least another cross-section adjacent to the selected at least one cross-section, and a synthesized image acquiring unit which acquires at least one synthesized 2D image by synthesizing the at least one first 2D image and the at least one second 2D image.

The display unit may display the synthesized 2D image and the acquired 3D image.

According to another aspect of the present invention, there is provided a method of displaying a 3D ultrasonic image and a 2D ultrasonic image, the method comprising: acquiring a 3D image of a target object; selecting at least one cross-section of the target object based on an external input for the acquired 3D image; acquiring a 2D image by scanning the target object such as to obtain the selected at least one cross-section; and displaying the 2D image and the 3D image.

The acquiring of the 3D image may comprise acquiring the 3D image of the target object by combining a plurality of pieces of image data that are acquired for each cycle of an ultrasound having a predetermined period.

The selecting of the at least one cross-section may comprise producing at least one window which is to be located on the acquired 3D image, and moving the at least one window produced on the 3D image.

The at least one cross-section may be selected from the 3D image of the target object so as to correspond to the location of the at least one window moved on the 3D image.

The acquiring of the 2D image may comprise acquiring at least one 2D image by scanning the target object in the scan direction of the selected at least one cross-section.

The displaying may comprise displaying the 2D image and the 3D image.

The selecting of the at least one cross-section may further comprise additionally selecting at least another cross-section adjacent to the selected at least one cross-section.

The adjacent at least one cross-section may be a cross-section that is a predetermined distance separated from the at least one cross-section selected from the 3D image.

The acquiring of the 2D image may comprise acquiring at least one first 2D image by scanning the target object such as to obtain the selected at least one cross-section, acquiring at least one second 2D image by scanning the target object such as to obtain the at least another cross-section adjacent to the selected at least one cross-section, and acquiring at least one synthesized 2D image by synthesizing the at least one first 2D image and the at least one second 2D image.

The displaying may comprise displaying the synthesized 2D image and the 3D image together.

According to another aspect of the present invention, there is provided a computer-readable recording medium having recorded thereon a program for executing the above-described method.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram of an apparatus for displaying a three-dimensional (3D) ultrasonic image and a two-dimensional (2D) ultrasonic image, according to an embodiment of the present invention;
FIG. 2A illustrates an example of 3D image acquisition;
FIG. 2B illustrates 3D image acquisition according to an embodiment of the present invention;
FIG. 3 is a block diagram of a cross-section selection unit included in the apparatus illustrated in FIG. 1, according to an embodiment of the present invention;
FIG. 4 illustrates 2D image acquisition according to an embodiment of the present invention;
FIG. 5 is a block diagram of an apparatus for displaying a 3D ultrasonic image and a 2D ultrasonic image, according to another embodiment of the present invention;
FIG. 6 illustrates acquisition of a synthesized 2D image according to an embodiment of the present invention;
FIG. 7 is a flowchart of a method of displaying a 3D ultrasonic image and a 2D ultrasonic image, according to an embodiment of the present invention; and
FIG. 8 is a flowchart of a method of displaying a 3D ultrasonic image and a 2D ultrasonic image, according to another embodiment of the present invention.

### DETAILED DESCRIPTION OF THE INVENTION

Terminology used herein will now be briefly described, and the present invention will be described in detail.

Although general terms being widely used at present were selected as terminology used in the present invention while considering the functions of the present invention, they may vary according to intentions of one of ordinary skill in the art, judicial precedents, the advent of new technologies, and the like. Terms arbitrarily selected by the applicant of the present invention may also be used in a specific case. In this case, their meanings need to be given in the detailed description of the present invention. Hence, the terms must be defined based on the meanings of the terms and the contents of the entire specification, not by simply stating the terms themselves.

It will be understood that the terms "comprises" and/or "comprising" or "includes" and/or "including" when used in this specification, specify the presence of stated elements, but do not preclude the presence or addition of one or more other elements. Terms such as "... unit" and "module" stated in the specification denote units that process at least one function or operation, and they may be implemented by using hardware, software, or a combination of hardware and software.

In the entire specification, an "ultrasonic image" denotes an image of a target object that is acquired using ultrasounds. The target object may denote a part of a human body. For example, the target object may be an organ, such as a liver, a heart, a womb, a brain, a breast, or an abdomen, or a fetus.

In the entire specification, a "user" may be a medical expert, such as a doctor, a nurse, a medical technologist, or a medical image expert, but the present invention is not limited thereto.

The present invention will now be described more fully with reference to the accompanying drawings, in which exemplary embodiments of the invention are shown. The invention may, however, be embodied in many different forms and should not be construed as being limited to the embodiments set forth herein. In the drawings, parts irrelevant to the description are omitted for simplicity of explanation, and like numbers refer to like elements throughout.

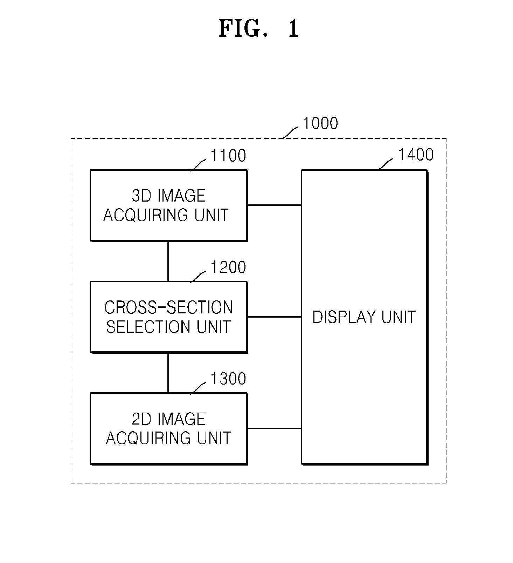

FIG. 1 is a block diagram of an apparatus 1000 for displaying a three-dimensional (3D) ultrasonic image and a two-dimensional (2D) ultrasonic image, according to an embodiment of the present invention.

Referring to FIG. 1, the apparatus 1000 may include a 3D image acquiring unit 1100, which acquires a 3D image of a target object, a cross-section selection unit 1200, which selects at least one cross-section of the target object based on an external input for the acquired 3D image, a 2D image acquiring unit 1300, which acquires a 2D image by scanning the target object such as to obtain the selected cross-section, and a display unit 1400, which displays the 2D image and the 3D image.

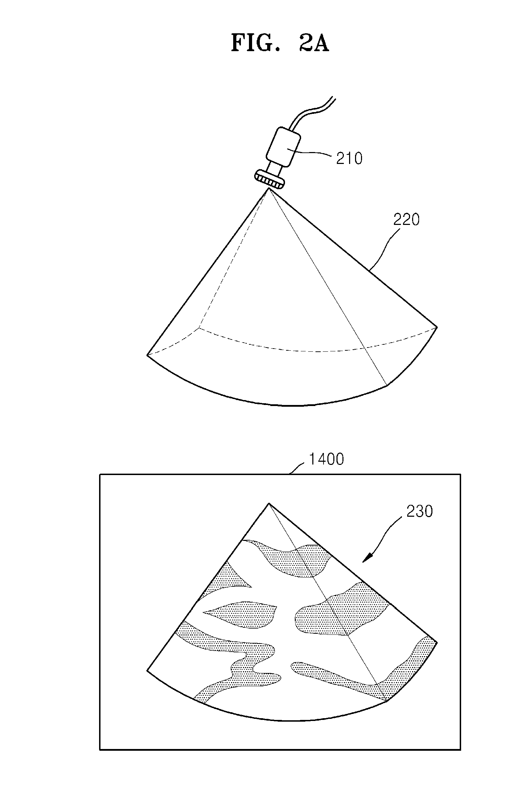

FIG. 2A illustrates an example of 3D image acquisition.

For example, the 3D image acquiring unit 1100 may radiate an ultrasound to the target object within a predetermined region 220 of a probe 210 and may acquire a 3D image 230 in response to an echo signal of the radiated ultrasound. Such a 3D image may include an image that voluminously expresses the whole or a part of the target object.

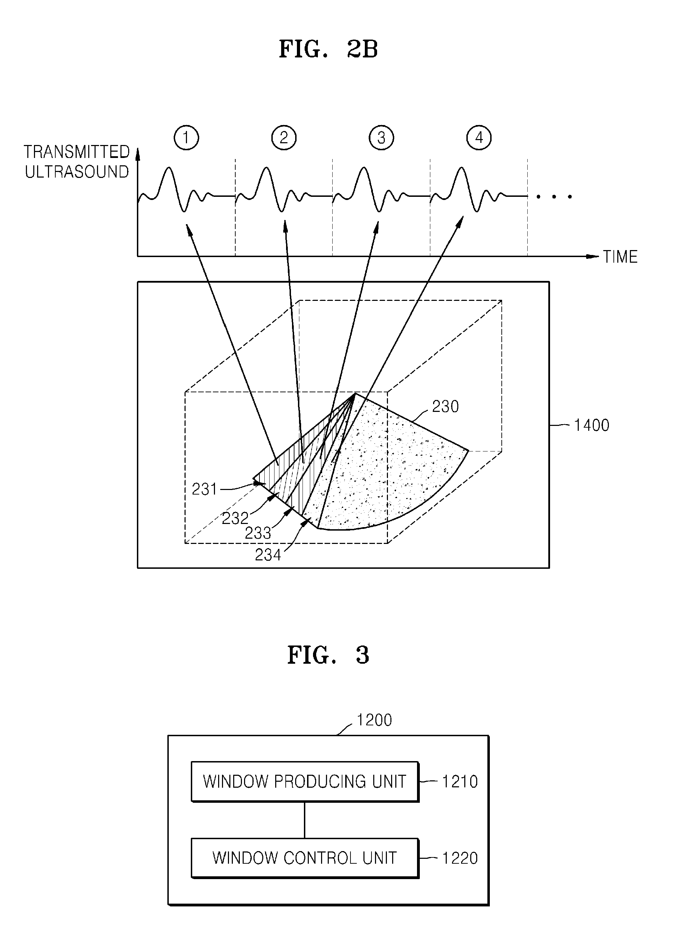

FIG. 2B illustrates acquisition of the 3D image 230 according to an embodiment of the present invention.

The 3D image acquiring unit 1100 may acquire the 3D image 230 of the target object by combining a plurality of pieces of image data that are acquired for each cycle of an ultrasound having a predetermined period.

For example, as illustrated in FIG. 2B, the 3D image 230 of the target object may be acquired by combining a plurality of pieces of image data acquired for each cycle of an ultrasonic signal having a predetermined period. For example, an ultrasonic image 231 of a first part may be acquired from an echo signal corresponding to a section of a first cycle of an ultrasonic signal transmitted toward a target object, namely, a first section ①. An ultrasonic image 232 of a second part may be acquired from an echo signal corresponding to a second section (②) of the ultrasonic signal. Similarly, an ultrasonic image 233 of a third part and an ultrasonic image 234 of a fourth part may be respectively acquired from an echo signal corresponding to a third section ③ of the ultrasonic signal and an echo signal corresponding to a fourth section ④ of the ultrasonic signal.

The acquired ultrasonic images 231 through 234, namely, a plurality of acquired pieces of image data, may be combined to generate a combined image, by using a conventional image matching technique or the like. The combined image may include the 3D image 230 for the whole or a part of the target object, as illustrated in FIG. 2B.

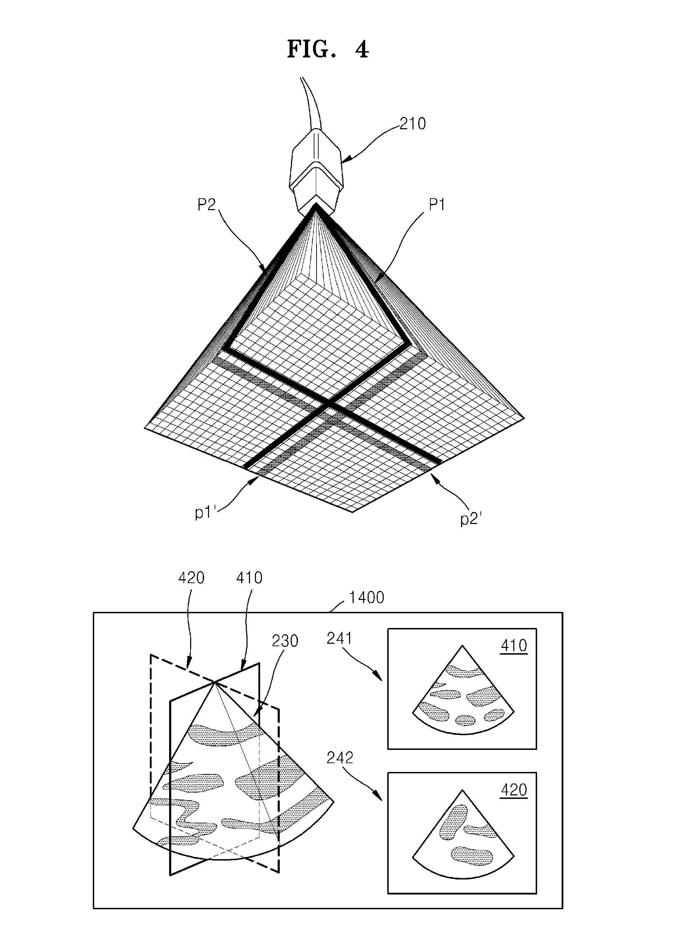

FIG. 3 is a block diagram of the cross-section selection unit 1200 of FIG. 1, according to an embodiment of the present invention. FIG. 4 illustrates 2D image acquisition according to an embodiment of the present invention.

Referring to FIG. 3, the cross-section selection unit 1200 may include a window producing unit 1210, which produces at least one window which is to be located on the acquired 3D image 230, and a window control unit 1220, which moves the window produced on the 3D image 230.

At least one cross-section may be selected from the 3D image 230 by the cross-section selection unit 1200 so as to correspond to the location of at least one window, namely, windows 410 and 420, moved on the 3D image 230.

For example, referring to FIG. 4, at least one cross-section may be selected using the windows 410 and 420, which are movable on the 3D image 230 captured from the target object. The at least one cross-section may include a cross-section that includes a region of the target object that a user wants to observe with interest via a 2D image. In other words, the at least one cross-section may include a cross-section for acquiring a 2D image of the target object.

As illustrated in FIG. 4, the windows 410 and 420 may be moved on the 3D image 230 by a control signal applied to the window control unit 1220, based on an external input signal received from a user input unit (not shown). Then, at least one cross-section that traverses the target object may be determined by the movable windows 410 and 420, and the determined cross-sections may be selected by the cross-section selection unit 1200 to serve as cross-sections for acquiring 2D images.

The 2D image acquiring unit 1300 may acquire at least one 2D image by scanning the target object in the scan directions of the selected cross-sections.

As illustrated in FIG. 4, the 2D image acquiring unit 1300 may acquire at least one 2D image, namely, 2D images 241 and 242, by scanning the target object in scan directions P1 and P2 of the cross-sections selected by the cross-section selection unit 1200. The 3D image 230 may comprise a 3D ultrasound image, and the 2D images 241 and 242 may comprise 2D ultrasound images.

In other words, the 2D images 241 and 242 may be acquired by scanning the target object in radiation directions corresponding to the selected cross-sections (for example, the scan directions P1 and P2) from among directions in which an ultrasonic signal is radiated within a predetermined range by a probe 210 toward the target object. For example, the 2D image 241 may be acquired by scanning the target object in a scan direction (for example, the scan direction P1) corresponding to the cross-section determined by the windows 410. Similarly, the 2D image 242 may be acquired by scanning the target object in the scan direction P2.

The display unit 1400 may display the acquired 2D image and the acquired 3D image together. For example, as illustrated in FIG. 4, the 2D images 241 and 242 acquired by scanning the target object in the directions corresponding to the selected cross-sections may be displayed together with the 3D image 230, on the display unit 1400. In other words, the 2D images 241 and 242 and the 3D image 230 may be simultaneously displayed on the display unit 1400.

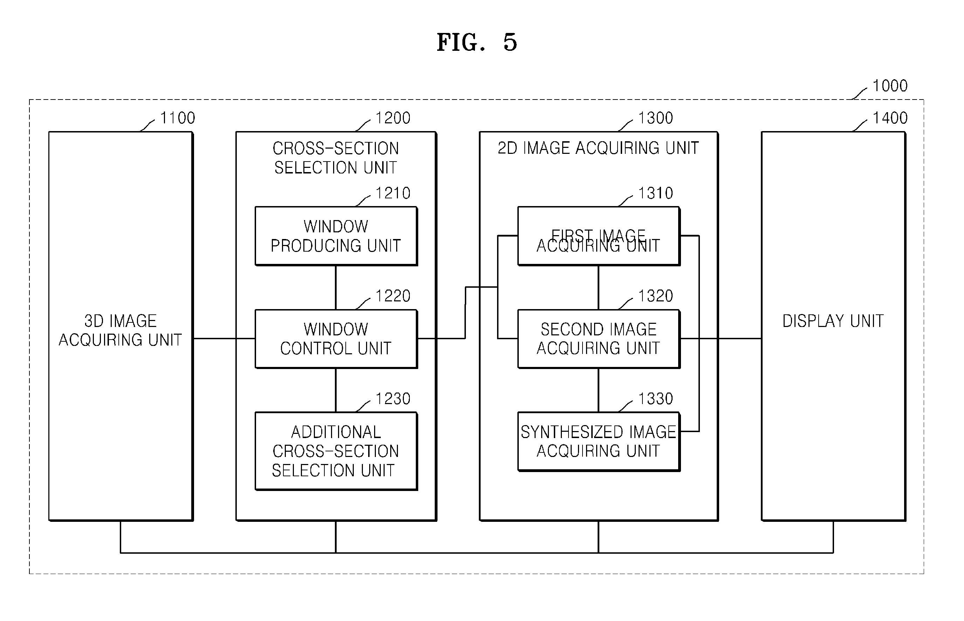

FIG. 5 is a block diagram of an apparatus 1000 for displaying a 3D ultrasonic image and a 2D ultrasonic image, according to another embodiment of the present invention.

Referring to FIG. 5, the cross-section selection unit 1200 may further include an additional cross-section selection unit 1230, which additionally selects at least another cross-section adjacent to the selected one cross-section, in addition to the window producing unit 1210 and the window control unit 1220 of FIG. 3. The adjacent one cross-section may include a cross-section that is a predetermined distance separated from the cross-section selected from the 3D image.

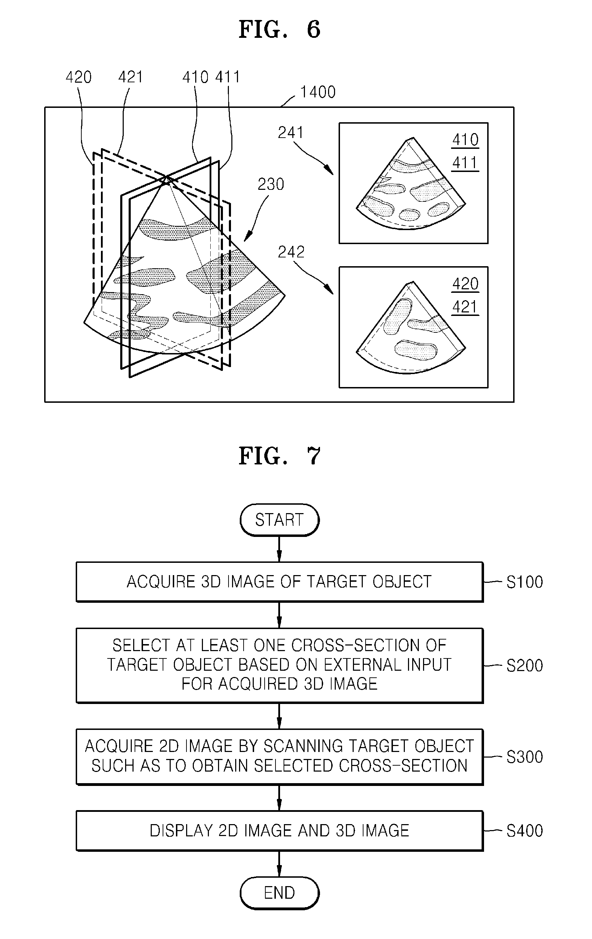

FIG. 6 illustrates acquisition of a synthesized 2D image according to an embodiment of the present invention.

When the cross-sections are determined and selected based on the windows 410 and 420 by the window control unit 1220, the cross-section selection unit 1200 may additionally select cross-sections adjacent to the selected at least one cross-section.

Referring to FIG. 6, when cross-sections based on the windows 410 and 420 are determined, at least one window, namely, windows 411 and 421, separated from the determined cross-sections by a predetermined distance may be additionally produced by the window producing unit 1210. At least one 2D image may be acquired by scanning the target object, as described above, in the scan directions corresponding to cross-sections determined by the additional windows 411 and 421.

Referring to FIG. 5, the 2D image acquiring unit 1300 may include a first image acquiring unit 1310, which acquires at least one first 2D image by scanning the target object such as to obtain the selected cross-section, a second image acquiring unit 1320, which acquires at least one second 2D image by scanning the target object such as to obtain at least one cross-section adjacent to the selected cross-section, and a synthesized image acquiring unit 1330, which acquires at least one synthesized 2D image by synthesizing the first 2D image and the second 2D image.

For example, as illustrated in FIG. 4, the first 2D images may be acquired by scanning the target object in ultrasound radiation directions, namely, the scan directions P1 and P2, corresponding to the windows 410 and 420. The second 2D images may be acquired by scanning the target object in ultrasound scan directions P1' and P2' corresponding to the additional windows 411 and 421, which are respectively adjacent to the windows 410 and 420.

The synthesized image acquiring unit 1330 may acquire the synthesized 2D image by synthesizing the acquired first 2D image and the acquired second 2D image by using a conventional image synthesizing technique or the like. For example, a voluminous 2D image 241 may be acquired by synthesizing the first and second 2D images which have been respectively acquired by scanning the target object in the scan directions corresponding to the windows 410 and 411. Similarly, a voluminous 2D image 242 may be acquired by synthesizing images which have been respectively acquired by scanning the target object in the scan directions corresponding to the windows 420 and 421. The voluminous 2D images 241 and 242 may be referred to as synthesized 2D images.

The display unit 1400 may display such a synthesized 2D image and a 3D image together. In other words, as illustrated in FIG. 6, the voluminous 2D images 241 and 242 and the 3D image 230 may be simultaneously displayed on the display unit 1400.

FIG. 7 is a flowchart of a method of displaying a 3D image 230 and a 2D image 241 or 242, according to an embodiment of the present invention.

Referring to FIG. 7, the method may include operation S100 of acquiring a 3D image of a target object, operation S200 of selecting at least one cross-section of the target object based on an external input for the acquired 3D image, operation S300 of acquiring a 2D image by scanning the target object such as to obtain the selected cross-section, and operation S400 of displaying the 2D image and the 3D image.

Operation S100 may include acquiring the 3D image 230 of the target object by combining a plurality of pieces of image data that are acquired for each cycle of an ultrasound having a predetermined period.

Operation S200 may include the operations of producing at least one window which is to be located on the acquired 3D image 230 and moving the window produced on the 3D image 230.

The at least one cross-section may be selected from the 3D image 230 of the target object by the cross-section selection unit 1200 so as to correspond to the location of the at least one window moved on the 3D image 230.

Operation S300 may include acquiring at least one 2D image by scanning the target object in the scan direction of the selected cross-section.

Operation S400 may include displaying the acquired 2D image and the acquired 3D image 230 together.

FIG. 9 is a flowchart of a method of displaying a 3D ultrasonic image and a 2D ultrasonic image, according to another embodiment of the present invention.

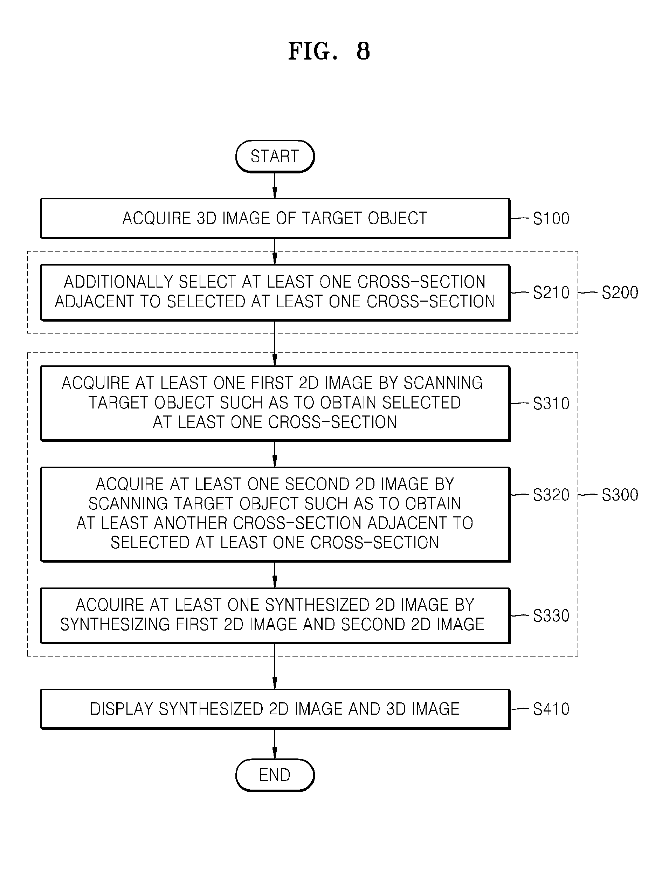

Referring to FIG. 8, operation S200 may further include operation S21 0 of additionally selecting at least one cross-section adjacent to the selected cross-section, in addition to the producing of the at least one window and the moving of the produced window of FIG. 7. The adjacent cross-section may include a cross-section that is a predetermined distance separated from the cross-section selected from the 3D image 230.

Operation S300 may include operation S310 of acquiring at least one first 2D image by scanning the target object such as to obtain the selected cross-section, operation S320 of acquiring at least one second 2D image by scanning the target object such as to obtain at least one cross-section adjacent to the selected cross-section, and operation S330 of acquiring at least one synthesized 2D image by synthesizing the first 2D image and the second 2D image.

In operation S41 0, the synthesized 2D image and the acquired 3D image are displayed together.

The contents of the above-described apparatuses of FIGS. 1 and 5 may be equally applied to the methods of FIGS. 7 and 8. Accordingly, descriptions of the above-described apparatuses 1000 of FIGS. 1 and 5 related to the methods of FIGS. 7 and 8 will not be repeated here.

The above-described embodiments of the present invention may be written as computer programs and may be implemented in general-use digital computers that execute the programs using a computer readable recording medium.

Examples of the computer readable recording medium include magnetic storage media (e.g., ROM, floppy disks, hard disks, etc.), optical recording media (e.g., CD-ROMs, or DVDs), etc.

Up to now, the present invention has been described by referring to exemplary embodiments. While the exemplary embodiments have been particularly shown and described, it will be understood by those of ordinary skill in the art that various changes in form and details may be made therein without departing from the spirit and scope of the present invention as defined by the appended claims. Therefore, the exemplary embodiments should be considered in descriptive sense only and not for purposes of limitation. Therefore, the scope of the present invention is defined not by the detailed description of exemplary embodiments, but by the appended claims, and all differences within the scope will be construed as being included in the present invention.

## Claims

1. An apparatus for displaying a three-dimensional (3D) ultrasonic image and a two-dimensional (2D) ultrasonic image, the apparatus comprising:
a 3D image acquiring unit which acquires a 3D image of a target object;
a cross-section selection unit which selects at least one cross-section of the target object based on an external input for the acquired 3D image;
a 2D image acquiring unit which acquires a 2D image by scanning the target object such as to obtain the selected at least one cross-section; and
a display unit which displays the 2D image and the 3D image.

2. The apparatus of claim 1, wherein the 3D image acquiring unit acquires the 3D image of the target object by combining a plurality of pieces of image data that are acquired for each cycle of an ultrasound having a predetermined period.

3. The apparatus of claim 1, wherein
the cross-section selection unit comprises:
a window producing unit which produces at least one window which is to be located on the acquired 3D image; and
a window control unit which moves the at least one window produced on the 3D image, and
the at least one cross-section is selected from the 3D image of the target object by the cross-section selection unit so as to correspond to the location of the at least one window moved on the 3D image.

4. The apparatus of claim 1, wherein the 2D image acquiring unit acquires at least one 2D image by scanning the target object in the scan direction of the selected at least one cross-section.

5. The apparatus of claim 4, wherein the display unit displays the 2D image and the 3D image.

6. The apparatus of claim 1, wherein
the cross-section selection unit further comprises an additional cross-section selection unit which additionally selects at least another cross-section adjacent to the selected at least one cross-section, and
the adjacent at least one cross-section is a cross-section that is a predetermined distance separated from the at least one cross-section selected from the 3D image.

7. The apparatus of claim 6, wherein the 2D image acquiring unit comprises:
a first image acquiring unit which acquires at least one first 2D image by scanning the target object such as to obtain the selected at least one cross-section;
a second image acquiring unit which acquires at least one second 2D image by scanning the target object such as to obtain the at least another cross-section adjacent to the selected at least one cross-section; and
a synthesized image acquiring unit which acquires at least one synthesized 2D image by synthesizing the at least one first 2D image and the at least one second 2D image.

8. The apparatus of claim 7, wherein the display unit displays the synthesized 2D image and the acquired 3D image.

9. A method of displaying a 3D ultrasonic image and a 2D ultrasonic image, the method comprising:
acquiring a 3D image of a target object;
selecting at least one cross-section of the target object based on an external input for the acquired 3D image;
acquiring a 2D image by scanning the target object such as to obtain the selected at least one cross-section; and
displaying the 2D image and the 3D image.

10. The method of claim 9, wherein the acquiring of the 3D image comprises acquiring the 3D image of the target object by combining a plurality of pieces of image data that are acquired for each cycle of an ultrasound having a predetermined period.

11. The method of claim 9, wherein
the selecting of the at least one cross-section comprises:
producing at least one window which is to be located on the acquired 3D image; and
moving the at least one window produced on the 3D image, and
the at least one cross-section is selected from the 3D image of the target object so as to correspond to the location of the at least one window moved on the 3D image.

12. The method of claim 9, wherein the acquiring of the 2D image comprises acquiring at least one 2D image by scanning the target object in the scan direction of the selected at least one cross-section.

13. The method of claim 12, wherein the displaying comprises displaying the 2D image and the 3D image.

14. The method of claim 9, wherein
the selecting of the at least one cross-section further comprises additionally selecting at least another cross-section adjacent to the selected at least one cross-section, and
the adjacent at least one cross-section is a cross-section that is a predetermined distance separated from the at least one cross-section selected from the 3D image.

15. A non-transitory computer-readable recording medium having recorded thereon a program for executing the method of any of claims 9 through 14.
